# EUROPEAN PATENT SPECIFICATION

(11) **EP 0 496 709 B1**
(45) Date of publication and mention of the grant of the patent: **02.10.1996**
(21) Application number: 92830021.9
(22) Date of filing: 21.01.1992
(51) Int. Cl.: A61F 13/15

(54) **Disposable sanitary towel provided with improved lateral tabs and method of manufacture thereof**
Wegwerfbare Damenbinde mit verbesserten Seitenlappen und Methode zur Herstellung davon
Serviette hygiénique jetable à volets latéraux perfectionnés, et méthode pour sa production

(30) Priority: 25.01.1991 IT TO910048
(43) Date of publication of application: 29.07.1992
(73) Proprietor: ANGELINI RICERCHE S.P.A. - SOCIETA' CONSORTILE (or, briefly, "ANGELINI RICERCHE S.P.A."), 00040 Pomezia - Santa Palomba (Roma) (IT)
(72) Inventor: Palumbo, Gianfranco, 65125 Pescara (IT)
(74) Representative: Bosotti, Luciano

(56) References cited:
- EP-A- 0 335 527
- FR-A- 2 644 694
- US-A- 4 285 343
- US-A- 4 764 242

## Description

The present invention relates to towels for feminine hygiene and relates more specifically to towels for feminine hygiene having lateral protection tabs which can be folded under the user's underwear.

Different types of articles intended for the absorbption of body fluids are known and available on the market.

In particular, with reference to towels for intimate feminine hygiene, two principal classes of products are known; namely external towels which are utilised to protect and cover the vulvar region of the user, being positioned within the underwear, and internal tampons which are introduced into the interior of the vaginal passage to intercept and absorb the menstrual flow.

External towels provided with lateral protection tabs are known; for example, in US Patent US-A-4285343 there is described a sanitary towel provided with lateral tabs and with an absorbent element of substantially rectangular form; the said lateral tabs may be integral or inserted and secured to the longitudinal edges of the towels themselves.

In use the towel, with the absorbent element located centrally with respect to the lateral panels, is positioned in the user's underwear and possibly fixed there, whilst the lateral panels are folded downwardly and fixed to the outer surface of the underwear in the region of the gusset.

In the above cited patent it is pointed out how such a structure acts so that the central absorbent element does not curve along the longitudinal axis but remains substantially flat or slightly concave conforming itself to the body surface.

Moreover this type of towel avoids staining of underwear and provides a greater absorbent capacity.

Nowadays anatomically shaped towels are becoming ever more wide spread, in which the towels central absorbent zone is narrower than the end zones; these are more agreeable from the aesthetic point of view and adapt better to the user's anatomy.

The solution proposed in the above cited United States patent, whilst being functional, has several disadvantages as far as industrial production is concerned, involving a greater use of raw materials and consequently higher costs than similar products without lateral tabs.

Naturally this is particularly disadvantageous for a disposable product.

Therefore there is a commercial necessity for absorbent products for intimate feminine hygiene provided with lateral protection tabs, which do not involve the use of raw materials and therefore production costs which are significantly greater than those of towels which do not have lateral tabs. Specifically, the present invention relates to a method for manufacturing disposable sanitary towels having the features set forth in the preamble of Claim 1 as well as a towel according to the preamble of Claim 3. Exemplary of such sanitary towels is eg. the one shown in EP-A-0 335 527. From US-A-4 764 242 a method is known wherein sanitary products such as baby diapers or adult incontinent briefs are manufactured starting from a continuous strip of material and the outline of each product is cut from that strip.

It is an objective of the present invention to provide a sanitary towel for women, having lateral tabs which can be folded and fixed along the edges of the underwear in the region of the crotch , the cost of which will not be significantly greater than that of a towel having no such lateral tabs, obtaining this by means of a saving of materials which would otherwise be discarded.

It is a further objective of the present invention to provide a sanitary towel for women, having lateral tabs with a characteristic form which can be folded easily along the edges of the underwear.

According to the present invention these objectives are achieved by means of a method having the further characteristics set out in Claim 1 and a towel having the further characteristics set forth in Claim 3.

The invention will now be described, purely by way of non limitative example, with reference to the attached drawings, in which:
Figure 1 is a view of a sanitary towel formed according to the present invention, seen from the side intended to be opposite the user;
Figure 2 is a sectional view of the towel taken on the line II-II of Figure 1;
Figure 3 is a plan view of a continuous strip of sheet of material starting from which the constituent elements of a sanitary towel formed according to the present invention are obtained; and
Figure 4 is a plan view of a preferred alternative configuration of sanitary towel formed according to the present invention.

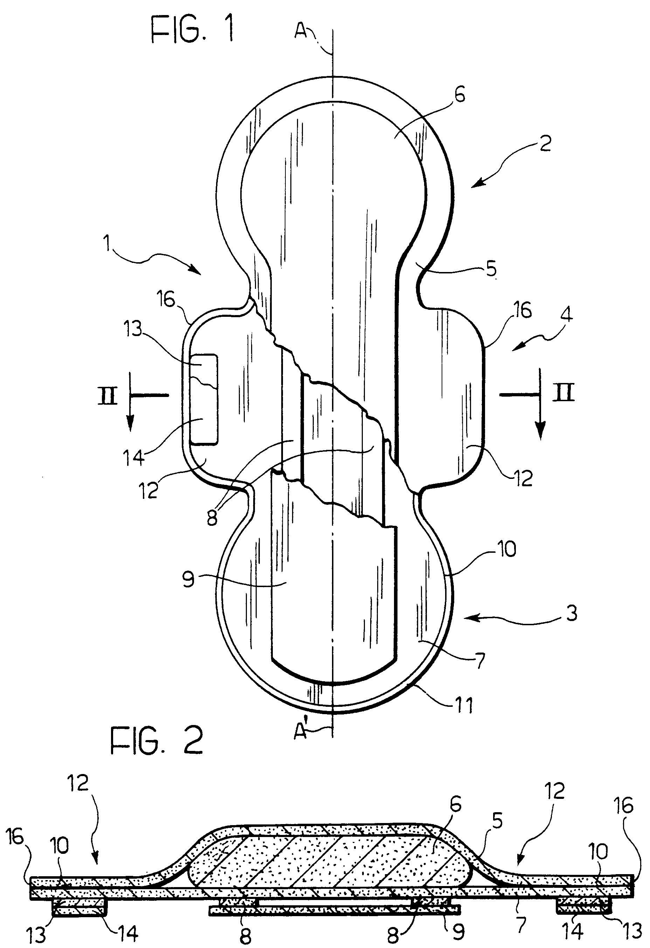

The present invention relates to disposable absorbent articles and more particularly to sanitary towels for women which are worn in direct contact with the outer body in the vulvar region of the user and serve the purpose of absorbing body fluids, being then thrown away after having been used once. The disposable sanitary towel shown in Figures 1 and 2 is a preferred embodiment of the absorbent article formed according to the present invention. In Figure 1 there is shown a sanitary towel 1 formed according to the present invention with a portion of the structure removed to illustrate the construction with greater clarity; in particular there is shown the lower side of the towel which, in use, comes into direct contact with the underwear.

In Figure 1 can be recognised a front region 2, a rear region 3 and a central region 4 lying between them; a longitudinal axis AA′ and a transverse axis corresponding to the section line II-II can also be seen.

The towel comprises a liquid-permeable layer 5, for example of non-woven fabric or perforated plastics, intended to come into direct contact with the user's body, an absorbent element 6 generally made of hydrophilic fibre, and an impermeable layer 7 of plastics.

The towel is preferably provided on the lower side with an adhesive region, generally disposed longitudinally, to be able to fix it to the interior of the underwear in the region of the crotch : in the illustrated configuration this adhesive zone is constituted by two parallel strips 8 of pressure-sensitive adhesive disposed symmetrically along the two sides of the longitudinal axis AA′ and protected by a layer silicone-treated paper 9 which can easily be removed by the user upon use.

The absorbent element 6 is shaped in such a way as to have a greater width at the front region 2 and the rear region 3 of the towel, being therefore narrower in the central region 4; this shape, as is known, allows a greater adaptability to the user's anatomy.

In the preferred configuration illustrated in Figure 1, the absorbent element 6 is shaped in such a way as to have two wider zones, situated in correspondence with the front regions 2 and the rear regions 3 of the towel, identical to one another and symmetrical with respect to the transverse axis II-II, whilst the narrower zone situated at the central region 4 of the towel has rectilinear edges parallel to the longitudinal axis AA′.

The liquid-permeable layer 5 and the impermeable layer 7 have the same shape and dimensions corresponding to the outer shape of the towel as a whole and are joined together, for example by means of a weld line or by adhesive 10 along at least the outer outline 11 of the towel.

As shown in Figures 1 and 2 the liquid-permeable layer 5 and the impermeable layer 7 extend on both sides of the absorbent element 6, in the central region 4 of the towel itself, to form two tabs 12 intended in use to be folded downwardly and fixed to the outer surface of the underwear in the crotch region thanks to two pressure-sensitive adhesive zones 13 present on the lower side of the said tabs and covered with silicone-treated paper 14 which is peeled off upon use; in Figure 1 only one of the said zones 13 of pressure sensitive adhesive is illustrated.

As is known, during use, the said tabs are able to protect the longitudinal edges of the underwear in the crotch region.

A salient characteristic of the sanitary towel formed according to the present invention is provided by the presence of the lateral tabs 12 formed by making use of the portions of material which would otherwise be discarded to obtain a sanitary towel of the type shaped so as to be narrower in the central region 4.

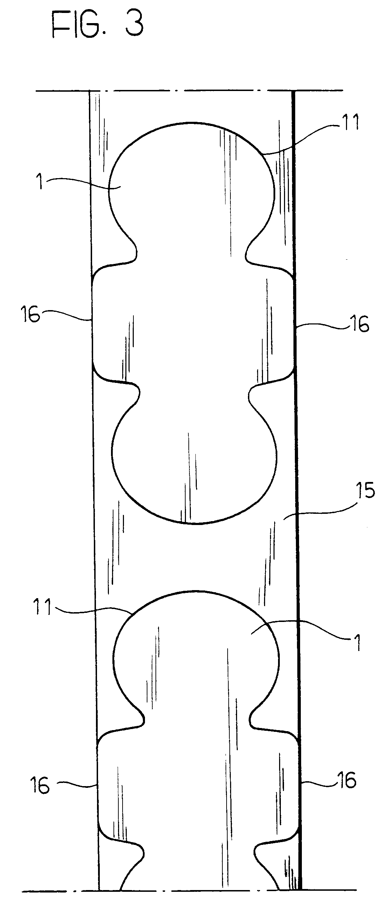

As shown in Figure 3 the towels 1 of the present invention are made in an entirely known way by cutting the outline 11 from a continuous strip 15 formed by two superimposed layers constituted by a liquid-permeable layer 5 and an impermeable layer 7 with a series of absorbent elements 6 interposed at regular intervals and disposed longitudinally and centred with respect to the longitudinal axis of the strip.

In general the width of the strip 15 is greater, for technical reasons, than the width of the sanitary towel which is to be formed from it; currently the width of the strip 15 exceeds the width of the sanitary towels by about 20ö25mm overall.

In the case of shaped sanitary towels this width of the strip must be greater than the maximum width of the sanitary towel at the front and rear regions 2 and 3.

According to the present invention the tabs 12 are formed in the central region 4 of the sanitary towel by utilising substantially the entire width of the strip 15; the lateral edges 16 of the said tabs will coincide at least partly, depending on the shape of the tabs themselves, with the longitudinal edges of the strip 15, apart from any possible misalignments of the edges of the two superimposed layers which form the strip itself.

In other words, when the two tabs 12 of the central region 4 are not folded they have an extent (that is to say the width measured along the line II-II) greater than the width, that is the dimension in the same direction, of the end regions 2, 3.

The lateral tabs 12 are therefore formed by utilising portions of the strip 15 which otherwise would be discarded and for the most part in regions where the waste of material would be greater because of the narrower width of the sanitary towel in comparison with the overall width of the strip.

For example, in the illustrated embodiment, the sanitary towel formed according to the present invention is formed from a continuous strip having a width of 125mm; the said towel has a length of 240mm and a width of 100mm in the front region 2 and rear region 3; at the central region 4 the width varies between a minimum of 65mm, where there are no tabs, and a maximum of 125mm across the tabs 12.

The saving of material for each individual towel produced will therefore consist of two bands of overall width equal to 60mm and of a length approximately equal to the longitudinal extent of the tabs themselves.

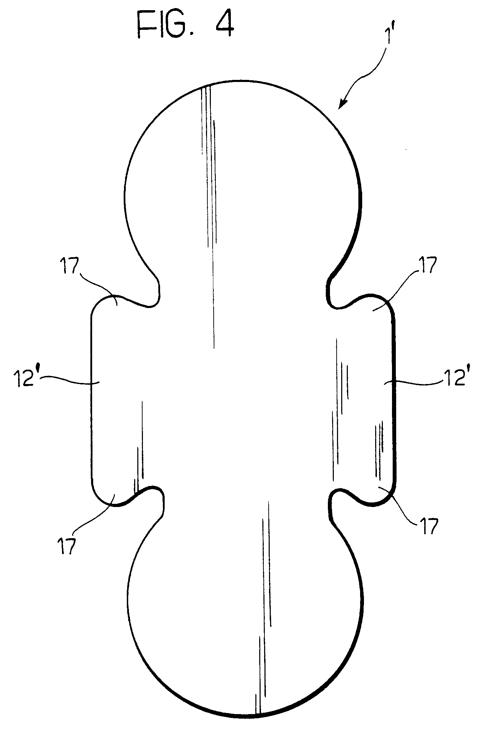

In Figure 4 there is shown an alternative preferred configuration of a sanitary towel 1′ formed according to the present invention; in this the tabs 12′ have a greater extent than in the case illustrated in Figure 3 by the effect of four lobes 17 which, whilst not having a specific practical function, improve the towel from the aesthetic point of view.

Naturally, the principle of the invention remaining the same, the details can be widely varied with respect to those described and illustrated; in particular continuous strip 15 of greater width than that illustrated can be used, and also the width of the central region 4 can be reduced.

Finally, it is to be noted that the sanitary towels formed according to the present invention have demonstrated, during closed tests, an excellent acceptance by the users thanks to the extremely comfortable retained shape, to the ease of positioning the tabs beneath the underwear and to the efficacy of the tabs themselves in preventing staining of the underwear.

## Claims

1. A method of manufacturing disposable sanitary towels (1) intended to be positioned, in use, within the user's underwear in correspondence with the crotch, comprising a liquid-permeable layer (5), an impermeable layer (7) and an absorbent element (6), provided with end regions (2, 3) as well as a narrower central region (4) extending from and joined to the said end regions (2, 3) and two tabs (12) which extend transversely from the said central region (4) and which, in use, can be folded under and fixed to the outer surface of the underwear, wherein said tabs (12) have respective lateral edges (16) defining the width of the sanitary towels, characterised in that:
- the said towels are formed by cutting the outline (11) thereof from a continuous strip (15) of material of predetermined width,
- said predetermined width of the continuous strip (15) is selected to correspond to said width of the sanitary towels; and
- said cutting is limited to said end regions (2, 3), whereby said lateral edges (16) of the tabs coincide, at least partly, with the longitudinal edges of said strip (15) and said tabs (12) are made by utilising substantially the entire width of said continuous strip (15).

2. A method according to claim 1, characterised by the fact that it includes the operation of providing adhesive regions (8) and (13) on the lower side of the said strip (15).

3. A disposable sanitary towel (1) intended to be positioned, in use, within the user's underwear in correspondence with the crotch, comprising a liquid-permeable layer (5), an impermeable layer (7) and an absorbent element (6), provided with end regions (2, 3) of predetermined width, a narrower central region (4) extending from and joined to the said end regions (2, 3) and two tabs (12) which extend transversely from the said central region (4) and which, in use, can be folded under and fixed to the outer surface of the underwear, wherein said tabs (12), when unfolded in the said central region (4) have respective lateral edges (16) which extend beyond the width of the said end regions (2, 3), characterized in that said narrower central region (4) has rectilinear edges parallel to the longitudinal axis of the towel and said lateral edges (16) of the tabs (12) are also rectilinear and parallel to said longitudinal axis.

4. A sanitary towel according to claim 3, characterised by the said fact that its lower side is provided with at least one longitudinally disposed adhesive zone (8) for securing the towel, in use, to the inner part of the user's underwear.

5. A sanitary towel according to any of claims 3 or 4, characterised by the fact that its lower side is provided with at least one adhesive zone (13) on each tab (12).

## Patentansprüche

1. Verfahren zur Herstellung von Wegwerfdamenbinden (1), die bei Benützung zum Einlegen in die Unterwäsche der Trägerin dem Schritt entsprechend vorgesehen sind, mit einer flüssigkeitsdurchlässigen Schicht (5), einer undurchlässigen Schicht (7) und einem absorbierenden Element (6), umfassend zwei Endbereiche (2, 3) sowie einen schmäleren, mittleren Bereich (4), der sich von den Endbereichen (2, 3) aus erstreckt und mit diesen verbunden ist, und zwei Laschen (12), welche sich in Querrichtung von dem mittleren Bereich (4) aus erstrecken und bei Benützung nach unten gebogen und an der Außenfläche der Unterwäsche fixiert werden können, wobei die Laschen (12) jeweils Seitenränder (16) haben, die die Breite der Damenbinden begrenzen, dadurch gekennzeichnet, daß
- die genannten Binden durch Ausschneiden ihrer Umrisse (11) aus einem durchgehenden Streifen (15) aus Material mit bestimmter Breite gebildet werden,
- die bestimmte Breite des durchgehenden Streifens (15) so gewählt wird, daß er der Breite der Damenbinden entspricht; und
- das Ausschneiden auf die Endbereiche (2, 3) begrenzt ist, wobei die Seitenränder (16) der Laschen zumindest teilweise mit den Längsrändern des Streifens (15) zusammenfallen, und die Laschen (12) durch Ausnützen der im wesentlichen gesamten Breite des durchgehenden Streifens (15) ausgebildet werden.

2. Verfahren nach Anspruch 1, dadurch gekennzeichnet, daß es das Anbringen von Haftbereichen (8) und (13) an der Unterseite des Streifens (15) umfaßt.

3. Wegwertdamenbinde (1), die bei Benützung zum Einlegen in die Unterwäsche der Trägerin dem Schritt entsprechend vorgesehen ist, mit einer flüssigkeitsdurchlässigen Schicht (5), einer undurchlässigen Schicht (7) und einem absorbierenden Element (6), umfassend zwei Endbereiche (2, 3) bestimmter Breite, einen schmäleren mittleren Bereich (4), der sich von den Endbereichen (2, 3) aus erstreckt und mit diesen verbunden ist, und zwei Laschen (12), welche sich in Querrichtung vom mittleren Bereich (4) aus erstrecken und bei Benützung nach unten gebogen und an der Außenfläche der Unterwäsche fixiert werden können, wobei die Laschen (12), wenn sie vom mittleren Bereich (4) herausgeklappt werden, jeweils Seitenränder (16) haben, die sich über die Breite der Endbereiche (2, 3) hinaus erstrecken, dadurch gekennzeichnet, daß der schmälere mittlere Bereich (4) geradlinige Ränder hat, die sich parallel zur Längsachse der Binde erstrecken, und daß die Seitenränder (16) der Laschen (12) auch geradlinig sind und parallel zur genannten Längsachse verlaufen.

4. Damenbinde nach Anspruch 3, dadurch gekennzeichnet, daß ihre Unterseite mit mindestens einem in Längsrichtung angeordneten Haftbereich (8) versehen ist, der bei Benützung zur Befestigung der Binde an der Innenseite der Unterwäsche der Trägerin bestimmt ist.

5. Damenbinde nach einem der Ansprüche 3 oder 4, dadurch gekennzeichnet, daß ihre Unterseite mit mindestens einem Haftbereich (13) auf jeder Lasche (12) versehen ist.

## Revendications

1. Procédé de fabrication de serviettes hygiénique jetables (1) destinées à être placées, lors de l'utilisation, à l'intérieur du sous-vêtement de l'utilisatrice au niveau de l'entrejambe, comprenant une couche perméable aux liquides (5), une couche imperméable (7) et un élément absorbant (6), avec des zones d'extrémité (2,3) ainsi qu'avec une zone centrale plus étroite (4) s'étendant depuis lesdites zones d'extrémité (2,3) et reliée à ces dernières et deux languettes (12) qui s'étendent transversalement
depuis ladite zone centrale (4) et qui, lors de l'utilisation, peuvent être pliées sous et fixées à la surface extérieure du sous-vêtement, les languettes (12) ayant des bords latéraux respectifs (16) définissant la largeur des serviettes hygiéniques, caractérisé en ce que :
- lesdites serviettes sont formées par coupe de leur contour (11) à partir d'une bande continue (15) de matériau de largeur prédéterminée,
- ladite largeur prédéterminée de la bande continue (15) est choisie de façon à correspondre à ladite largeur des serviettes hygiéniques; et
- ladite coupe est limitée auxdites zones d'extrémité (2,3), par quoi lesdits bords latéraux (16) des languettes coïncident, au moins partiellement, avec les bords longitudinaux de ladite bande (15) et lesdites languettes (12) sont fabriquées en utilisant sensiblement la largeur totale de ladite bande continue (15).

2. Procédé selon la revendication 1, caractérisé par le fait qu'il comprend l'opération de réalisation de zones adhésives (8) et (13) sur le côté inférieur de ladite bande (15).

3. Serviette périodique jetable (1) destinée à être mise en place, lors de l'utilisation, à l'intérieur du sous-vêtement de l'utilisatrice au niveau de l'entrejambe, comprenant une couche perméable aux liquides (5), une couche imperméable (7) et un élément absorbant (6), avec des zones d'extrémité (2,3) de largeur prédéterminée, une zone centrale plus étroite (4) s'étendant depuis les zones d'extrémité (2,3) et reliée à ces dernières et deux languettes (12) qui s'étendent transversalement depuis ladite zone centrale (4) et qui, lors de l'utilisation, peuvent être pliées sous et fixées à la surface extérieure du sous-vêtement, dans laquelle lesdites languettes (12), quand elles sont déployées depuis ladite zone centrale (4) ont des bords latéraux respectifs (16) qui se prolongent au delà de la largeur desdites zones d'extrémité (2,3), caractérisée en ce que ladite zone centrale plus étroite (4) a des bords rectilignes parallèles à l'axe longitudinal de la serviette et lesdits bords latéraux (16) des languettes (12) sont également rectilignes et parallèles audit axe longitudinal.

4. Serviette hygiénique selon la revendication 3, caractérisée par le fait que son côté inférieur est muni d'au moins une zone adhésive (8) disposée longitudinalement pour fixer la serviette, lors de l'utilisation, à la partie intérieure du sous-vêtement de l'utilisatrice.

5. Serviette hygiénique selon l'une quelconque des revendications 3 et 4, caractérisée par le fait que son côté inférieur est muni d'au moins une zone adhésive (13) sur chaque languette (12).
